# EUROPEAN PATENT APPLICATION

(11) **EP 4 687 134 A1**
(43) Date of publication of application: **04.02.2026**
(21) Application number: 25185593.8
(22) Date of filing: 26.06.2025
(51) Int. Cl.: G09G 3/3233, G06F 3/041

(54) **DISPLAY DEVICE INCLUDING OPTICAL SENSING CIRCUIT AND ELECTRONIC DEVICE INCLUDING THE DISPLAY DEVICE**

(30) Priority: 31.07.2024 KR 20240101422
(71) Applicant: Samsung Display Co., Ltd., Yongin-si, Gyeonggi-do 17113 (KR)
(72) Inventor: MOON, Gyeong-Ub, Yongin-si (KR); AN, Jongyeop, Yongin-si (KR); YANG, Dongwook, Yongin-si (KR); LEE, Hyeon Jun, Yongin-si (KR)
(74) Representative: Marks & Clerk LLP

(57) **Abstract**

A display device comprises a display panel including pixel circuits and optical sensing circuits, a gate driver configured to provide gate signals to the pixel circuits and the sensing circuits, a readout circuit receiving sensing currents output from the optical sensing circuits in response to the gate signals and generating a pulse signal based on the sensing currents, and a driving controller configured to control the gate driver and the readout circuit, and to determine a user's a biometric indicator based on the pulse signal. The optical sensing circuits initialized with a reset voltage in response to reset signals are grouped into first to n-th groups. The reset signals are sequentially applied to the first to n-th groups as first to n-th group reset signals during n frames, wherein n is an integer greater than or equal to 2.

## Description

### BACKGROUND

### 1. Field

Embodiments of the present inventive concept relate to a display device including an optical sensing circuit and an electronic device including the display device.

### 2. Description of the Related Art

Recently, an electronic device performing biosensing has been developed. The biosensing may be performed by generating a sensing current in response to a reflected light generated from a user's body by an optical sensing circuit of the electronic device, and generating a digital sensing signal based on the sensing current by a readout circuit of the electronic device.

The optical sensing circuit may be formed in an external manner or an embedded manner. In an optical sensing circuit of an embedded manner, the light sensing circuit exists separately from a display device. In an optical sensing circuit of an embedded manner, the optical sensing circuit exists inside the display device. In general, a smartwatch may be an electronic device of the external manner, and a smartphone may be an electronic device of the embedded manner. The electronic device of the external manner may require a light exposure period of microseconds, and the electronic device of the embedded manner may require a light exposure period of milliseconds.

In some embodiments, in order for the biosensing to be performed accurately, it may be important to sufficiently secure the light exposure period and a frequency of the digital sensing signal.

### SUMMARY

Embodiments of the present inventive concept provide a display device for sufficiently securing a frequency of a light exposure period and a digital sensing signal.

Embodiments of the present inventive concept provide an electronic device including the display device.

In an embodiment of a display device according to the present inventive concept, the display device comprises a display panel including pixel circuits and optical sensing circuits, a gate driver configured to provide gate signals to the pixel circuits and the optical sensing circuits, a readout circuit receiving sensing currents output from the optical sensing circuits in response to the gate signals and generating a pulse signal based on the sensing currents, and a driving controller configured to control the gate driver and the readout circuit, and to determine a user's a biometric indicator based on the pulse signal. The optical sensing circuits initialized with a reset voltage in response to reset signals are grouped into first to n-th groups. The reset signals are sequentially applied to the first to n-th groups as first to n-th group reset signals during n frames, wherein n is an integer greater than or equal to 2.

In an embodiment, the display panel may include a display region in which the pixel circuits emit light and a sensing region in which the optical sensing circuits receive light that is reflected by the user, and the sensing region is the display region.

In an embodiment, during the n frames, the gate signals may be sequentially applied to the first to n-th groups as the first to n-th group gate signals, and during a k-th frame, the gate signals constituting a k-th group gate signal may be sequentially applied to optical sensing circuits included in a k-th group, wherein k is an integer greater than or equal to 1 and less than or equal to n.

In an embodiment, during the n frames, first to n-th group sensing currents may be generated as the sensing currents in response to the gate signals, and during the n frame, the pulse signal may be generated based on the first to n-th group sensing currents.

In an embodiment, a frequency of the pulse signal may be equal to a driving frequency of the display panel.

In an embodiment, in the k-th frame, reset signals constituting the k-th group reset signal may be simultaneously applied to the optical sensing circuits included in the k-th group.

In an embodiment, reset signal lines connected to the optical sensing circuits of the k-th group and transmitting the k-th group reset signal may be connected to each other.

In an embodiment, a light exposure period in which the optical sensing circuits receive a reflected light from the user may be determined based on a k-th group reset signal and a k-th group gate signal, and a light exposure period of the k-th group may be greater than n-1 frames and less than or equal to n frames.

In an embodiment, the display device may further comprises first to n-th control transistors connected to the first to n-th groups and controlled based on a reset control signal output from the driving controller, and the first to n-th control transistors may sequentially provide the reset signals to the first to n-th groups as the first to n-th group reset signals in response to the reset control signal.

In an embodiment, the display device may further comprises a demultiplexer connected to the first to n-th groups and controlled based on a reset control signal output from the driving controller, and the demux may sequentially provides the reset signals to the first to n-th groups as the first to n-th group reset signals in response to the reset control signal.

In an embodiment, the first to n-th groups may be grouped in a block line type shape or a dot type shape.

In an embodiment, the first to n-th group reset signals may be equal to the first to n-th group gate signals, and during the k-th frame, the reset signals constituting the k-th group reset signal may be sequentially applied to the optical sensing circuits in the k-th group.

In an embodiment, when the optical sensing circuit outputs the sensing current in response to the i-th gate signal, the optical sensing circuit may receive the i+n-th gate signal as the reset signal, wherein i is an integer greater than or equal to 2.

In an embodiment, the light exposure period during which the optical sensing circuits receive a reflected light from the user may be determined based on the k-th group reset signal and the k-th group gate signal, and the light exposure period is n frames long.

In an embodiment, a light exposure period during which the optical sensing circuits receive a reflected light from the user may be determined based on a k-th group reset signal and the k-th group gate signal, and an amplitude of the pulse signal increases with light exposure period.

In an embodiment, when a frequency of the pulse signal is high, a graph of the pulse signal may appear thick include a high number of jagged portions.

In an embodiment, the readout circuit may comprise an integrator including an inverting input terminal connected to the readout line, a non-inverting input terminal receiving a reference voltage, and an output terminal, and an integrating capacitor connected between the inverting input terminal of the amplifier and the output terminal of the amplifier, and integrating the sensing current to generate a signal voltage, a signal capacitor accumulating and storing the signal voltage, and an analog-to-digital converter connected to the signal capacitor.

In an embodiment, the display device may further comprise a data driver configured to provide a data voltage to the pixel circuits, and each of the pixel circuits may comprise a first pixel transistor which generates a driving current, a second pixel transistor which provides the data voltage to the first pixel transistor in response to a data write gate signal, and a light emitting element which emits light based on the driving current.

In an embodiment, the display device may further comprise an emission driver configured to provide an emission signal to the pixel circuits, each of the pixel circuits may further comprise a third pixel transistor diode-connecting the first pixel transistor in response to a compensation gate signal, a fourth pixel transistor providing a first initialization voltage to a gate electrode of the first pixel transistor in response to a data initialization gate signal, fifth and sixth pixel transistors applying the driving current to the light emitting element in response to the emission signal, and a seventh pixel transistor providing a second initialization voltage to a first electrode of the light emitting element in response to a light emitting element initialization gate signal, and each of the optical sensing circuits may comprise a first sensing transistor which generates the sensing current based on a voltage of a first electrode of the sensing element, a second sensing transistor which provides the sensing current to the readout line in response to the data write gate signal, and a third sensing transistor which provides the reset voltage to the first electrode of the sensing element in response to the reset signal.

In an embodiment of an electronic device according to the present inventive concept, the electronic device comprises a display panel including pixel circuits and optical sensing circuits, a gate driver configured to provide gate signals to the pixel circuits and the sensing circuits, a readout circuit receiving sensing currents output from the optical sensing circuits in response to the gate signals and generating a pulse signal based on the sensing currents, a driving controller configured to control the gate driver and the readout circuit, and to determine a user's a biometric indicator based on the pulse signal, and one or more processors configured to control the driving controller. The optical sensing circuits initialized with a reset voltage in response to reset signals are grouped to include first to n-th groups, and the reset signals are sequentially applied to the first to n-th groups as first to n-th group reset signals during n frames, wherein n is an integer greater than or equal to 2.

According to the display device and the electronic device, optical sensing circuits may be grouped to form first to n-th groups, and during n frame, reset signals may be sequentially applied to the first to n-th groups as first to n-th group reset signals. Accordingly, a light exposure period may be sufficiently secured, the light exposure period EIT may be constant at upper and lower portions of a display panel, and a frequency of a digital sensing signal (i.e., a pulse signal) may be sufficiently secured. Therefore, a consistency and a reliability of the digital sensing signal may be secured. That is, an amplitude of the pulse signal may be large, and the pulse signal may be smooth.

At least some of the above and other features of the invention are set out in the claims.

### BRIEF DESCRIPTION OF THE DRAWINGS

The above and other features of embodiments of the present inventive concept will become more apparent by describing in detailed embodiments thereof with reference to the accompanying drawings, in which:
FIG. 1 is a block diagram showing a display device according to embodiments of the present inventive concept;
FIG. 2 is a diagram showing an image of a fingerprint;
FIG. 3 is a diagram showing a pulse signal;
FIG. 4 is a circuit diagram showing an example of a pixel circuit and an optical sensing circuit of FIG. 1;
FIG. 5 is a timing diagram showing an example of signals applied to a pixel circuit of FIG. 4;
FIG. 6 is a timing diagram showing an example of signals applied to an optical sensing circuit of FIG. 4;
FIG. 7 is a circuit diagram showing an example of a readout circuit and optical sensing circuits of FIG. 1;.
FIG. 8 is a diagram showing a display panel of FIG. 1;
FIG. 9 is a diagram showing a sensing region of FIG. 8 that is touched by a user's a body;
FIG. 10 is a timing diagram showing a comparative example of signals applied to optical sensing circuits of FIG. 1;
FIG. 11 is a timing diagram showing a comparative example of signals applied to optical sensing circuits of FIG. 1, a sensing current generated by light sensing signals of FIG. 1, and a digital sensing signal generated by a readout circuit of FIG. 1;
FIG. 12 is a diagram showing an image of a fingerprint according to a light exposure period of FIG. 11;
FIG. 13 is a diagram showing a pulse signal according to a light exposure period of FIG. 11;
FIG. 14 is a timing diagram showing a comparative example of signals applied to optical sensing circuits of FIG. 1, a sensing current generated by optical sensing signals of FIG. 1, and a digital sensing signal generated by a readout circuit of FIG. 1;
FIG. 15 is a diagram showing a pulse signal according to a frequency of a digital sensing signal of FIG. 14;
FIG. 16 is a diagram showing grouped optical sensing circuits of FIG. 1 according to embodiments of the present inventive concept;
FIG. 17 is a diagram showing an example of a driving controller and optical sensing circuits of FIG. 16;
FIG. 18 is a diagram showing an example of a driving controller and optical sensing circuits of FIG. 16;
FIG. 19 is a timing diagram showing an example of signals applied to optical sensing circuits of FIG. 16 and FIG. 17, sensing currents generated by the optical sensing circuits of FIG. 16 and FIG. 17, and a digital sensing signal generated by a readout circuit of FIG. 1;
FIG. 20 is a diagram showing a form of readout lines connected to optical sensing circuits of FIG. 16 and FIG. 17;
FIG. 21 is a diagram showing optical sensing circuits of FIG. 16 and data write gate signals and reset signals applied to the optical sensing circuits of FIG. 16;
FIG. 22 is a timing diagram showing an example of signals applied to optical sensing circuits of FIG. 21, sensing currents generated by the optical sensing circuits of FIG. 21, and a digital sensing signal generated by a readout circuit of FIG. 1;
FIG. 23 is a block diagram showing an electronic device; and
FIG. 24 is a diagram showing an embodiment in which an electronic device of FIG. 23 is implemented as a smart phone.

### DETAILED DESCRIPTION OF THE EMBODIMENTS

Hereinafter, the present inventive concept will be described in more detail with reference to the accompanying drawings.

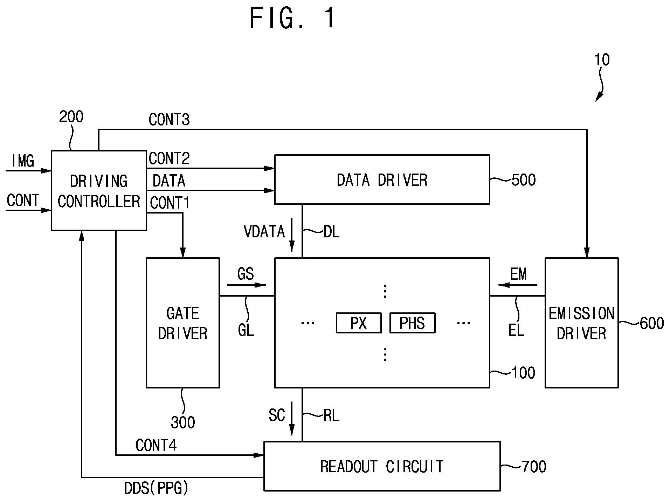

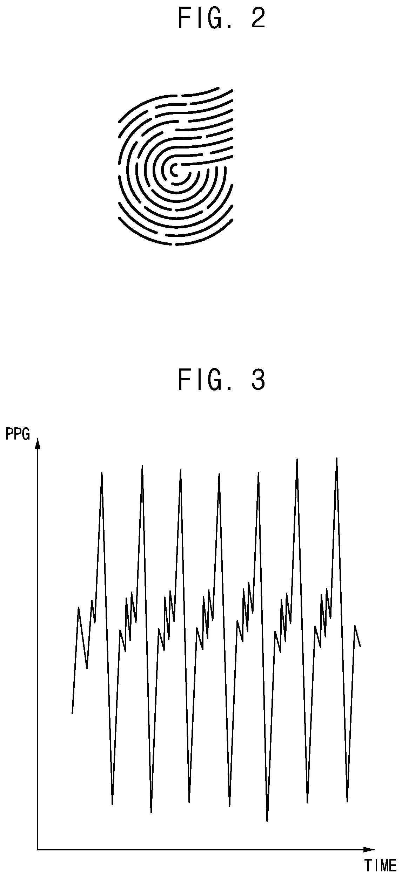

FIG. 1 is a block diagram showing a display device according to embodiments of the present inventive concept. FIG. 2 is a diagram showing an image of a fingerprint. FIG. 3 is a diagram showing a pulse signal.

Referring to FIGS. 1 to 3, a display device 10 may include a display panel 100 and a display panel driver. The display panel driver may include a driving controller 200, a gate driver 300, a data driver 500, an emission driver 600, and a readout circuit 700.

The display panel 100 may include a display region for displaying an image and a peripheral region arranged adjacent to the display region.

The display panel 100 may include pixel circuits PX connected to gate lines GL, data lines DL, and emission lines EL, and optical sensing circuits PHS connected to the gate lines GL and readout lines RL.

The pixel circuit PX may have a light emitting element, and the optical sensing circuit PHS may have a sensing element. For example, the light emitting element may be a light emitting diode. For example, the light emitting element may be an organic light emitting diode. For example, the light emitting element may be a quantum dot light emitting diode. For example, the sensing element may be a photodiode. For example, the sensing element may be an organic photodiode.

The driving controller 200 may receive input image data IMG and an input control signal CONT from an external device. For example, the input image data IMG may include red image data, green image data, and blue image data. The input image data IMG may include white image data. The input image data IMG may include magenta image data, yellow image data, and cyan image data. The input control signal CONT may include a master clock signal and a data enable signal. The input control signal CONT may further include a vertical synchronization signal and a horizontal synchronization signal.

The driving controller 200 may generate a first control signal CONT1, a second control signal CONT2, a third control signal CONT3, a fourth control signal CONT4, and a data signal DATA based on the input image data IMG and the input control signal CONT.

The driving controller 200 may generate the first control signal CONT1 for controlling an operation of the gate driver 300 based on the input control signal CONT, and output the first control signal CONT1 to the gate driver 300. The first control signal CONT1 may include a vertical start signal and a gate clock signal.

The driving controller 200 may generate the second control signal CONT2 for controlling an operation of the data driver 500 based on the input control signal CONT, and output the second control signal CONT2 to the data driver 500. The second control signal CONT2 may include a horizontal start signal and a load signal.

The driving controller 200 may generate the data signal DATA based on the input image data IMG. The driving controller 200 may output the data signal DATA to the data driver 500.

The driving controller 200 may generate the third control signal CONT3 for controlling an operation of the emission driver 600 based on the input control signal CONT, and output the third control signal CONT3 to the emission driver 600.

The driving controller 200 may generate the fourth control signal CONT4 for controlling an operation of the readout circuit 700 based on the input control signal CONT and output the fourth control signal CONT4 to the readout circuit 700.

The gate driver 300 may generate gate signals GS for driving the gate lines GL in response to the first control signal CONT1 received from the driving controller 200. The gate driver 300 may sequentially output the gate signals GS to the gate lines GL in units of rows.

The data driver 500 may receive the second control signal CONT2 and the data signal DATA from the driving controller 200, and convert the data signal DATA into a data voltage VDATA having an analog type. The data driver 500 may output the data voltage VDATA to the data line DL.

The emission driver 600 may generate emission signals EM for driving the emission lines EL in response to the third control signal CONT3 received from the driving controller 200. The emission driver 600 may sequentially output the emission signals EM to the emission lines EL in rows.

In FIG. 1, for convenience of explanation, the gate driver 300 may be arranged on a first side of the display panel 100 and the emission driver 600 may be arranged on a second side of the display panel 100. However, the present inventive concept is not limited to the arrangement that is explicitly described herein. For example, both the gate driver 300 and the emission driver 600 may be arranged on the first side of the display panel 100. For example, both the gate driver 300 and the emission driver 600 may be arranged on respective sides of the display panel 100. For example, the gate driver 300 and the emission driver 600 may be formed integrally.

The readout circuit 700 may perform a sensing operation (i.e., a bio-sensing operation) in response to the fourth control signal CONT4 received from the driving controller 200. The readout circuit 700 may sequentially receive sensing currents SC output from the optical sensing circuits PHS through the readout lines RL in units of rows, and may generate a digital sensing signal DDS (also referred to as DSS) based on the sensing currents SC. The readout circuit 700 may output the digital sensing signal DDS to the driving controller 200. In an embodiment, the readout circuit 700 may be implemented as an integrated circuit, and the integrated circuit may be called a Read-Out Integrated Circuit (ROIC).

The digital sensing signal DDS may be used for fingerprint sensing, pulse sensing, etc. For example, when the digital sensing signal DDS is used for fingerprint sensing, the digital sensing signal DDS may be a fingerprint signal generated based on a reflected light generated from a user's a fingerprint, and the driving controller 200 may generate an image of the fingerprint based on the fingerprint signal. FIG. 2 shows an example of a fingerprint image. For example, when the digital sensing signal DDS is used for pulse sensing, the digital sensing signal DDS may be a pulse signal PPG generated based on light that is reflected off of a blood vessel of the user, and the driving controller 200 may determine a biomarker of the user (e.g. a biometric indicator) based on the pulse signal PPG. FIG. 3 shows an example of pulse signal PPG. For example, the biomarker may be blood pressure. The biomarker may be heart rate, stress level, cardiovascular health, respiratory rate, vascular age, and oxygen saturation, etc.

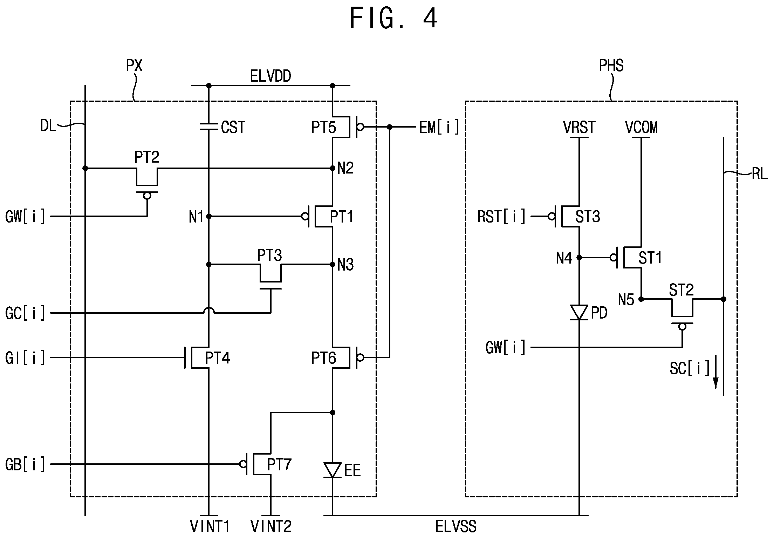

FIG. 4 is a circuit diagram showing an example of a pixel circuit and an optical sensing circuit of FIG. 1.

Referring to FIGS. 1 to 4, the pixel circuit PX may emit the light emitting element EE according to a level of the data voltage VDATA to display the image.

The pixel circuit PX may include first to seventh pixel transistors PT1 to PT7, a storage capacitor CST, and the light emitting element EE.

The first pixel transistor PT1 may include a gate electrode connected to a first node N1, a first electrode connected to a second node N2, and a second electrode connected to a third node N3.

The second pixel transistor PT2 may include a gate electrode receiving a data write gate signal GW, a first electrode receiving the data voltage VDATA, and a second electrode connected to the second node N2.

The third pixel transistor PT3 may include a gate electrode receiving a compensation gate signal GC, a first electrode connected to the first node N1, and a second electrode connected to the third node N3.

The fourth pixel transistor PT4 may include a gate electrode receiving a data initialization gate signal GI, a first electrode receiving a first initialization voltage VINT1, and a second electrode connected to the first node N1.

The fifth pixel transistor PT5 may include a gate electrode receiving an emission signal EM, a first electrode receiving a first power supply voltage ELVDD, and a second electrode connected to the second node N2.

The sixth pixel transistor PT6 may include a gate electrode receiving the emission signal EM, a first electrode connected to the third node N3, and a second electrode connected to a first electrode of the light emitting element EE.

The seventh pixel transistor PT7 may include a gate electrode receiving a light emitting element initialization gate signal GB, a first electrode receiving a second initialization voltage VINT2, and a second electrode connected to the first electrode of the light emitting element EE.

The storage capacitor CST may include a first electrode receiving the first power supply voltage ELVDD and a second electrode connected to the first node N1.

The light emitting element EE includes the first electrode and a second electrode receiving a second power supply voltage ELVSS.

For example, the first pixel transistor PT1, the second pixel transistor PT2, the fifth pixel transistor PT5, the sixth pixel transistor PT6, and the seventh pixel transistor PT7 may be P-type transistors. For example, the third pixel transistor PT3 and the fourth pixel transistor PT4 may be N-type transistors.

The optical sensing circuit PHS may generate the sensing current SC.

The optical sensing circuit PHS may include first sensing transistors ST1, a second sensing transistor ST2, a third sensing transistor ST3, and the sensing element PD.

The first sensing transistor ST1 may include a gate electrode connected to a fourth node N4, a first electrode receiving a first voltage VCOM, and a second electrode connected to a fifth node N5.

The second sensing transistor ST2 may include a gate electrode receiving the data write gate signal GW, a first electrode connected to the fifth node N5, and a second electrode connected to the readout line RL.

The third sensing transistor ST3 may include a gate electrode receiving a reset signal RST, a first electrode receiving a reset voltage VRST, and a second electrode connected to the fourth node N4.

The sensing element PD may include a first electrode connected to the fourth node N4 and a second electrode receiving the second power supply voltage ELVSS.

For example, the first, second, and third sensing transistors ST1, ST2, and ST3 may be P-type transistors.

In FIG. 4, the notation [i] may indicate that a signal is applied to an i-th row, and the data write gate signal GW[i], the compensation gate signal GC[i], the data initialization gate signal GI[i], the light emitting element initialization gate signal GB[i], the emission signal EM[i], and the reset signal RST[i] may be signals applied to the i-th row. As used herein, i is an integer greater than or equal to 1 and less than or equal to m.

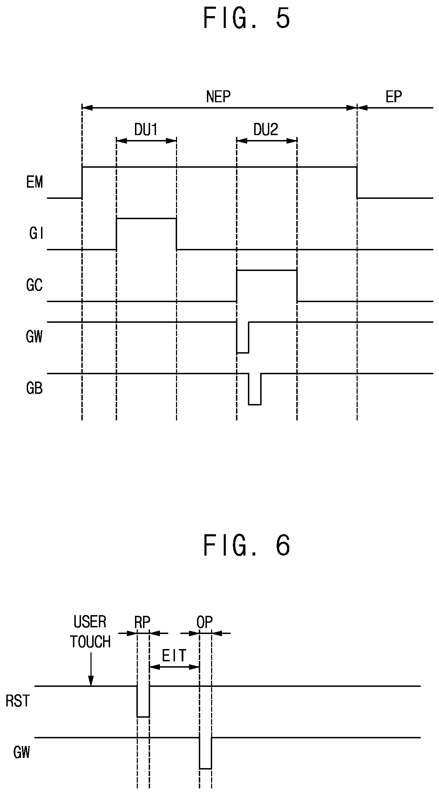

FIG. 5 is a timing diagram showing an example of signals applied to a pixel circuit of FIG. 4.

Referring to FIGS. 1 to 5, a driving period of the pixel circuit PX may include a non-emitting period NEP and an emitting period EP.

In the non-emitting period NEP, the fifth pixel transistor PT5 and the sixth pixel transistor PT6 may be turned off in response to an emission signal EM having a deactivation level, the driving current may not flow to the light emitting element EE, and the light emitting element EE may not emit light.

In the emitting period EP, the fifth pixel transistor PT5 and the sixth pixel transistor PT6 may be turned on in response to an emission signal EM having an activation level, the driving current may flow to the light emitting element EE, and the light emitting element EE may emit light.

The non-emitting period NEP may include a first period DU1 and a second period DU2.

In the first period DU1, the fourth pixel transistor PT4 may be turned on in response to a data initialization gate signal GI having an activation level, the first initialization voltage VINT1 may be applied to the first node N1, and the storage capacitor CST connected to the first node N1 may be initialized. In addition, the first pixel transistor PT1 may be turned on in response to the first initialization voltage VINT1 applied to the first node N1.

In the second period DU2, the second pixel transistor PT2 may be turned on in response to a data write gate signal GW having an activation level, and the data voltage VDATA may be applied to the second node N2. In addition, the third pixel transistor PT3 may be turned on in response to a compensation gate signal GC having an activation level, the first pixel transistor PT1 may be diode-connected, and a data voltage VDATA, whose threshold voltage of the first pixel transistor PT1 is compensated, may be applied to the first node N1 through the first pixel transistor PT1 and the third pixel transistor PT3. In addition, the seventh pixel transistor PT7 may be turned on in response to a light emitting element initialization gate signal GB having an activation level, and the second initialization voltage VINT2 may be applied to the first electrode of the light emitting element EE, and the light emitting element EE may be initialized. For example, a light emitting element initialization gate signal GB[i] of a current stage may be a data write gate signal GW[i+1] of a next stage.

In the emitting period EP, the first pixel transistor PT1 may be turned on in response to the data voltage VDATA and may generate the driving current. In addition, the fifth pixel transistor PT5 and the sixth pixel transistor PT6 may be turned on in response to an emission signal EM having an activation level, and the driving current may flow to the light emitting element EE through the fifth pixel transistor PT5, the first pixel transistor PT1, and the sixth pixel transistor PT6, and the light emitting element EE may emit the light based on the driving current.

The intensity of the driving current may be determined according to the level of the data voltage VDATA. The luminance of the light emitting element EE may be determined according to the intensity of the driving current.

A P-type transistor may be turned off in response to a high voltage level and turned on in response to a low voltage level. Since the first pixel transistor PT1, the second pixel transistor PT2, the fifth pixel transistor PT5, the sixth pixel transistor PT6, and the seventh pixel transistor PT7 are P-type transistors, an activation level of each of the emission signal EM, the data write gate signal GW, and the light emitting element initialization gate signal GB may be the low level, and a deactivation level of each of the emission signal EM, the data write gate signal GW, and the light emitting element initialization gate signal GB may be the high level.

An N-type transistor may be turned on in response to the high voltage level, and may be turned off in response to the low voltage level. Since the third pixel transistor PT3 and the fourth pixel transistor PT4 are N-type transistors, an activation level of each of the data initialization gate signal GI, and the compensation gate signal GC may be the high voltage level, and an inactivation level of each of the data initialization gate signal GI and the compensation gate signal GC may be the low voltage level.

FIG. 6 is a timing diagram showing an example of signals applied to an optical sensing circuit of FIG. 4.

Referring to FIGS. 1 to 6, a driving period of the optical sensing circuit PHS may include a reset period RP, a light exposure period EIT, and an output period OP. The light exposure period EIT may be a period in which photocharges are generated based on the reflected light generated from the user's body by the optical sensing circuits PHS. The reset period RP may be a period in which the optical sensing circuits PHS are initialized before the light exposure period EIT. The output period OP may be a period in which the sensing currents SC generated based on the generated photocharges are output.

The user's touch may occur on the display panel 100. In the reset period RP after the user's the touch, the third sensing transistor ST3 may be turned on in response to a reset signal RST having an activation level, the reset voltage VRST may be applied to the fourth node N4, and the sensing element PD may be initialized.

When the light emitting element EE emits light and the user's touch occurs on the display panel 100, light may be reflected off the user. In the light exposure period EIT, the sensing element PD may generate photocharges in response to the reflected light from the user, and a reverse current may be generated by the sensing element PD flowing from the sensing element PD to the fourth node N4. Therefore, as the light exposure period EIT increases, the photocharges may increase, the reverse current may increase, and a voltage of the fourth node N4 may increase. The first sensing transistor ST1 may be turned on in response to the voltage of the fourth node N4 and may generate the sensing current SC. The intensity of the sensing current SC may be determined according to a level of the voltage of the fourth node N4. That is, the intensity of the sensing current SC may be determined according to the light exposure period EIT.

In the output period OP, the second sensing transistor ST2 may be turned on in response to the data write gate signal GW having the activation level, and the sensing current SC may be output to the readout line RL.

As such, the reset period RP may be determined based on the reset signal RST, the light exposure period EIT may be determined based on the reset signal RST and the data write gate signal GW, and the output period OP may be determined based on the data write gate signal GW.

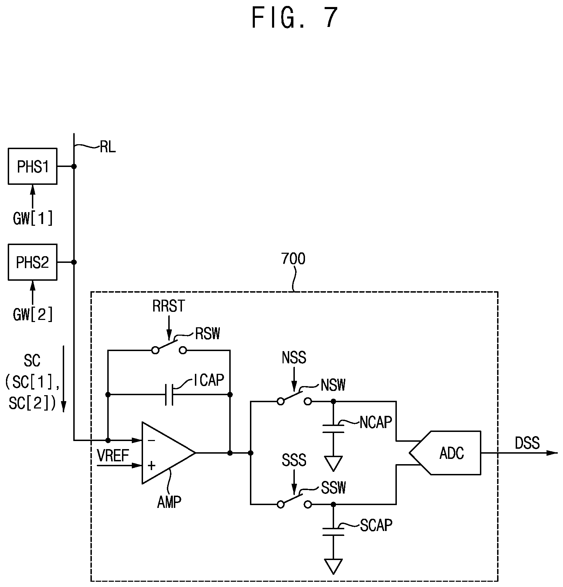

FIG. 7 is a circuit diagram showing an example of a readout circuit and optical sensing circuits of FIG. 1.

Referring to FIGS. 1 to 7, the readout circuit 700 may receive the sensing current SC of the optical sensing circuit PHS through the readout line RL, and may generate the digital sensing signal DSS based on the sensing current SC. The readout circuit 700 may include an amplifier AMP, an integrating capacitor ICAP, a noise capacitor NCAP, a signal capacitor SCAP, and an analog-to-digital converter ADC.

The amplifier AMP may include an inverting input terminal connected to the readout line RL, a non-inverting input terminal receiving a reference voltage VREF, and an output terminal. The integrating capacitor ICAP may be connected between the inverting input terminal of the amplifier AMP and the output terminal of the amplifier AMP.

The integrator composed of the amplifier AMP and the integrating capacitor ICAP may integrate the sensing current SC to generate a signal voltage and a noise voltage. The noise capacitor NCAP may store the noise voltage, and the signal capacitor SCAP may store the signal voltage and the noise voltage. In an embodiment, the readout line RL may be connected to light sensing rows PHS1, PHS2 to sequentially receive sensing currents SC[1], SC[2], the integrator may integrate the sensing currents SC[1], SC[2], and the signal capacitor SCAP may accumulate and store signal voltages generated based on the sensing currents SC[1], SC[2]. Accordingly, the amplitude of the digital sensing signal DSS may increase.

The analog-to-digital converter ADC may be connected to the noise capacitor NCAP and the signal capacitor SCAP. The analog-to-digital converter ADC may generate the digital sensing signal DSS based on a difference between a voltage stored in the signal capacitor SCAP and a voltage stored in the noise capacitor NCAP. Therefore, the analog-to-digital converter ADC may remove a component of the noise voltage included in the sensing current SC.

The readout circuit 700 may further include a reset switch RSW, a noise switch NSW, and a signal switch SSW.

The reset switch RSW may selectively connect the inverting input terminal of the amplifier AMP and the output terminal of the amplifier AMP in response to a readout reset signal RRST. The noise switch NSW may selectively connect the output terminal of the amplifier AMP and the noise capacitor NCAP in response to a noise switching signal NSS. The signal switch SSW may selectively connect the output terminal of the amplifier AMP and the signal capacitor SCAP in response to a signal switching signal SSS. In an embodiment, the reset signal RRST, the noise switching signal NSS, and the signal switching signal SSS may be included in the fourth control signal CONT4 of the driving controller 200.

As such, the sensing current SC may be converted into the digital sensing signal DSS. When the signal capacitor SCAP accumulates and stores the signal voltages, the amplitude of the digital sensing signal DSS may increase. As the light exposure period EIT increases, the intensity of the sensing current SC may increase, and the amplitude of the digital sensing signal DSS may increase.

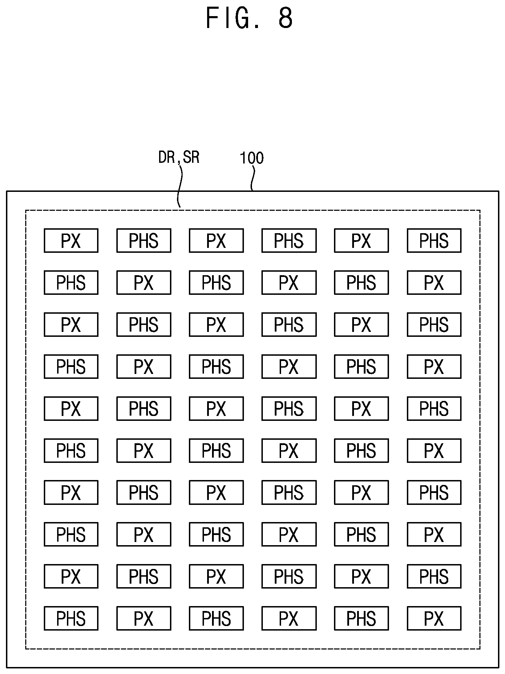

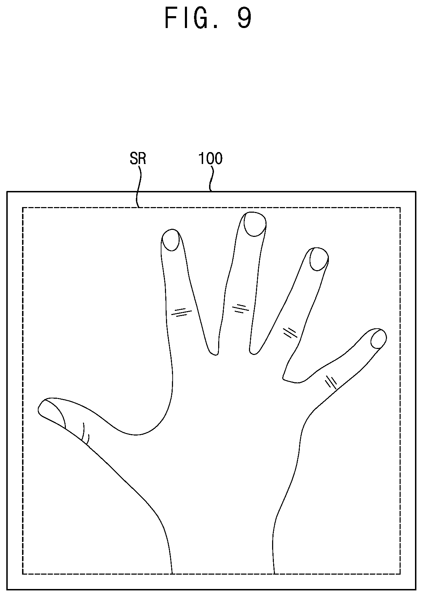

FIG. 8 is a diagram showing a display panel of FIG. 1. FIG. 9 is a diagram showing a sensing region of FIG. 8 that is touched by a user.

Referring to FIGS. 1 to 9, the display panel 100 may include the display region DR and a sensing region SR. The display region DR may be where the light emitting elements EE of the pixel circuits PX emit light. The sensing region SR may be where the sensing elements PD of the optical sensing circuits PHS receive the light reflected off the user's body.

As shown in FIG. 8, the pixel circuits PX and the optical sensing circuits PHS may be arranged adjacent to each other. Therefore, the sensing region SR is also the display region DR.

The sensing region SR according to embodiments of the present inventive concept may be the same area as the display region DR. However, the present inventive concept is not limited thereto. For example, the sensing region SR may be less than all of the display region DR (e.g., a lower central region).

In some embodiments, when the sensing region SR of the display panel 100 is large, a region of the user's body touching the display panel 100 may be large, such that a usability of the display device 10 including the optical sensing circuit PHS may increase. For example, the display panel 100 may be touched by the user's body (e.g., a palm as shown in FIG. 9).

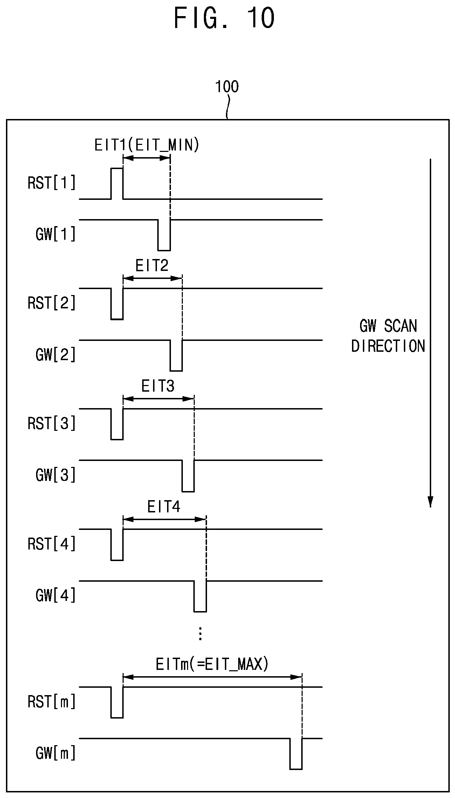

FIG. 10 is a timing diagram showing a comparative example of signals applied to optical sensing circuits of FIG. 1.

Referring to FIGS. 1 to 10, the optical sensing circuits PHS may configure first to m-th (where m is an integer greater than or equal to 2) light sensing rows. First to m-th data write gate signals GW[1], GW[2], GW[3], GW[4], ..., GW[m] and first to m-th reset signals RST[1], RST[2], RST[3], RST[4], ..., RST[m] may be applied to the first to m-th light sensing rows. For example, the first data write gate signal GW[1] and the first reset signal RST[1] may be applied to the first light sensing row. For example, the second data write gate signal GW[2] and the second reset signal RST[2] may be applied to the second light sensing row. For example, the third data write gate signal GW[3] and the third reset signal RST[3] may be applied to the third light sensing row. For example, the fourth data write gate signal GW[4] and the fourth reset signal RST[4] may be applied to the fourth light sensing row. For example, the m-th data write gate signal GW[m] and the m-th reset signal RST[m] may be applied to the m-th light sensing row.

Here, the first light sensing row may be arranged at an upper portion of the display panel 100, and the m-th light sensing row may be arranged at a lower portion of the display panel 100.

The first to m-th data write gate signals GW[1], GW[2], GW[3], GW[4], ..., GW[m] may be sequentially applied to the first to m-th light sensing rows, and the first to m-th reset signals RST[1], RST[2], RST[3], RST[4], ..., RST[m] may be simultaneously applied to the first to m-th light sensing rows. Therefore, first to m-th light exposure periods EIT1, EIT2, EIT3, EIT4, ..., EITm may gradually increase, with the first light exposure period EIT1 being a minimum light exposure period EIT_MIN, and the m-th light exposure period EITm being a maximum light exposure period EIT_MAX. As described above, as the light exposure period EIT increases, the amplitude of the digital sensing signal DSS may increase. Therefore, an amplitude of the digital sensing signal DSS of the upper portion of the display panel 100 may be small, and the amplitude of the digital sensing signal DSS of the lower portion of the display panel 100 may be large.

As such, when the amplitude of the digital sensing signal DSS is different according to a position of the display panel 100, consistency of the digital sensing signal DSS may be low.

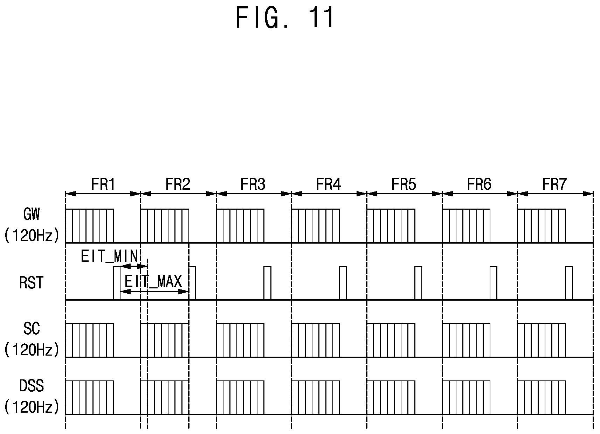

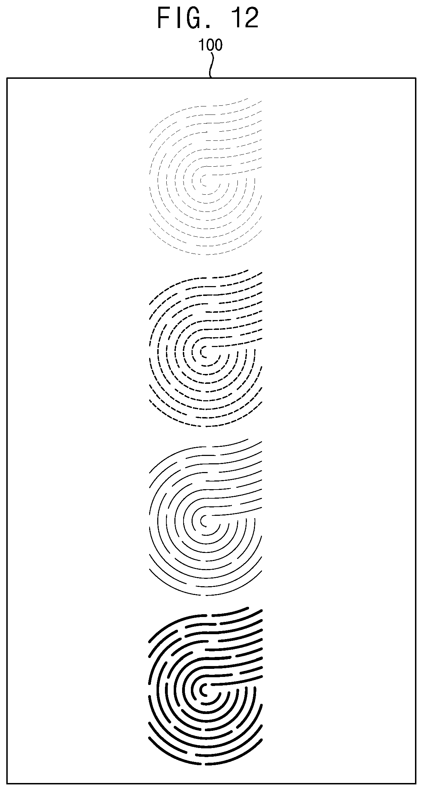

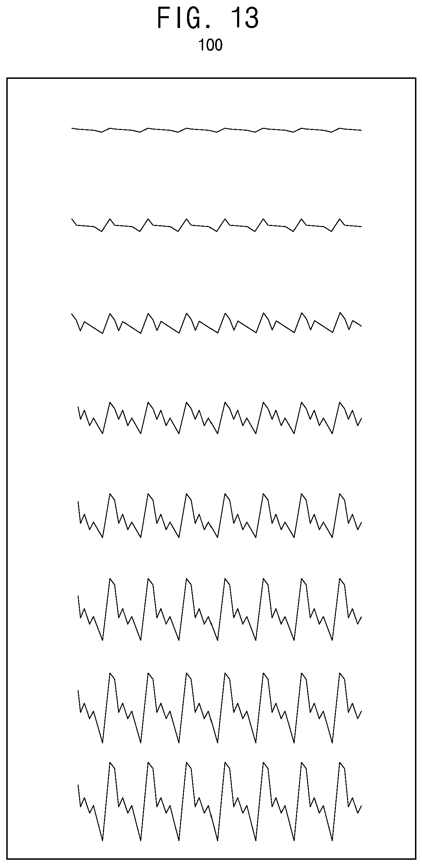

FIG. 11 is a timing diagram showing a comparative example of signals applied to optical sensing circuits of FIG. 1, a sensing current generated by light sensing signals of FIG. 1, and a digital sensing signal generated by a readout circuit of FIG. 1. FIG. 12 is a diagram showing an image of a fingerprint according to a light exposure period of FIG. 11. FIG. 13 is a diagram showing a pulse signal according to a light exposure period of FIG. 11.

Referring to FIGS. 1 to 13, a driving frequency of the display panel 100 may be 120 Hz, and a frequency of the data write gate signal GW may be 120 Hz. One frame of 120 Hz may be 8.33 ms. For example, in each of first to seventh frames FR1 to FR7, the first to m-th data write gate signals GW[1], GW[2], GW[3], GW[4], ..., GW[m] may be sequentially applied to the first to m-th light sensing rows. A frequency of the reset signal RST may be 120 Hz, and the first to m-th reset signals RST[1], RST[2], RST[3], RST[4], ..., RST[m] may be simultaneously applied to the first to m-th light sensing rows. In each of the first to m-th frames FR1 to FR7, the first to m-th data write gate signals GW[1], GW[2], GW[3], GW[4], ..., GW[m] may be sequentially applied to the first to m-th light sensing rows, and then the first to m-th reset signals RST[1], RST[2], RST[3], RST[4], ..., RST[m] may be simultaneously applied to the first to m-th light sensing rows.

Since the sensing current SC is generated based on the reset signal RST and the data write gate signal GW, and the frequency of the reset signal RST is 120 Hz, a frequency of the sensing current SC may be 120 Hz, and the frequency of the digital sensing signal DSS may be 120 Hz. In this case, for example, the minimum light exposure period EIT_MIN may be 0.38 ms, and the maximum light exposure period EIT_MAX may be 8.33 ms.

In some embodiments, 0.38 ms may be insufficient as the light exposure period EIT. For example, a light exposure period EIT greater than 8.33 ms may be used. As shown in FIG. 12, at the upper portion of the display panel 100, if the light exposure period EIT is short, the amplitude of the digital sensing signal DSS may be small, and the image of the fingerprint may be blurred. As shown in FIG. 13, at the upper portion of the display panel 100, if the light exposure period EIT is short, the amplitude of the digital sensing signal DSS may be small, and an amplitude of the pulse signal PPG may be small.

Therefore, to maintain consistency of the digital sensing signal DSS, the light exposure period EIT is set to be constant regardless of the position of the display panel 100. In order to secure the reliability of the digital sensing signal DSS, the light exposure period EIT is increased.

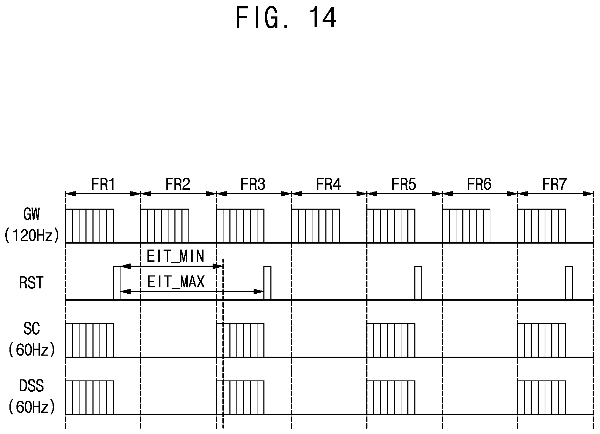

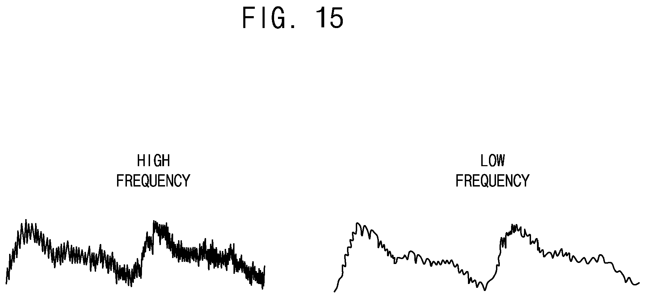

FIG. 14 is a timing diagram showing a comparative example of signals applied to optical sensing circuits of FIG. 1, a sensing current generated by optical sensing signals of FIG. 1, and a digital sensing signal generated by a readout circuit of FIG. 1. FIG. 15 is a diagram showing a pulse signal according to a frequency of a digital sensing signal of FIG. 14.

Referring to FIGS. 1 to 15, the driving frequency of the display panel 100 may be 120 Hz, and the frequency of the data write gate signal GW may be 120 Hz. One frame of 120 Hz may be 8.33 ms. For example, in each of the first, third, fifth, and seventh frames FR1, FR3, FR5, FR7, the first to m-th data write gate signals GW[1], GW[2], GW[3], GW[4], ..., GW[m] may be sequentially applied to the first to m-th light sensing rows. A frequency of the reset signal RST may be 60 Hz, and the first to m-th reset signals RST[1], RST[2], RST[3], RST[4], ..., RST[m] may be simultaneously applied to the first to m-th light sensing rows. In each of the first, third, fifth, and seventh frames FR1, FR3, FR5, FR7, the first to m-th data write gate signals GW[1], GW[2], GW[3], GW[4], ..., GW[m] may be sequentially applied to the first to m-th optical sensing rows, and then the first to m-th reset signals RST[1], RST[2], RST[3], RST[4], ..., RST[m] may be simultaneously applied to the first to m-th optical sensing rows.

Since the sensing current SC is generated based on the reset signal RST and the data write gate signal GW, and the frequency of the reset signal RST is 60 Hz, a frequency of the sensing current SC may be 60 Hz, and a frequency of the digital sensing signal DSS may be 60 Hz. In this case, for example, the minimum light exposure period EIT_MIN and the maximum light exposure period EIT_MAX may be longer than 8.33 ms.

In some embodiments, since the pulse signal PPG of the user changes over time, but an image of the fingerprint does not have the change over the time, the image of the fingerprint may be less affected by the frequency of the digital sensing signal DSS.

Sixty Hz may be small as the frequency of the digital sensing signal DSS (i.e., the pulse signal PPG). For example, a frequency of the digital sensing signal DSS greater than 100 Hz may be required. As shown in FIG. 15, when the frequency of the digital sensing signal DSS is low, the pulse signal PPG may be rougher than when the frequency is high. When the frequency is high, the pulse signal PPG become highly jagged as to appear as a thick line that is less rough.

In order to secure the reliability of the pulse signal PPG, the frequency of the digital sensing signal DSS needs to be large.

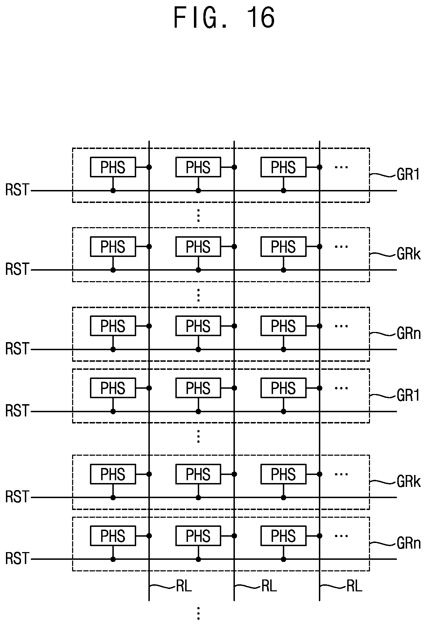

FIG. 16 is a diagram showing optical sensing circuits of FIG. 1 grouped into n groups according to embodiments of the present inventive concept.

Referring to FIGS. 1 to 16, optical sensing circuits PHS according to embodiments of the present inventive concept may be grouped to form first to n-th (where n is an integer of 2) groups GR1 to GRn. During n frames, reset signals RST may be sequentially applied to the first to n-th groups GR1 to GRn as first to n-th group reset signals.

For example, the reset signals RST may be applied as a first group reset signal to optical sensing circuits PHS of a first group GR1, the reset signals RST may be applied as a k-th group reset signal to optical sensing circuits PHS of a k-th group GRk (where k is an integer greater than or equal to 1 and less than or equal to n), and the reset signals RST may be applied as an n-th group reset signal to optical sensing circuits PHS of an n-th group GRn.

During the n frames, data write gate signals GW may be sequentially applied to the first to n-th groups GR1 to GRn as first to n-th group data write gate signals. During a k-th frame, reset signals constituting a k-th group data write gate signal may be sequentially applied to optical sensing circuits included in the k-th group GRk.

During the n frames, first to n-th group sensing currents may be generated as the sensing currents SC in response to the data write gate signals GW. During the n frames, the pulse signal PPG may be generated based on the first to n-th group sensing currents.

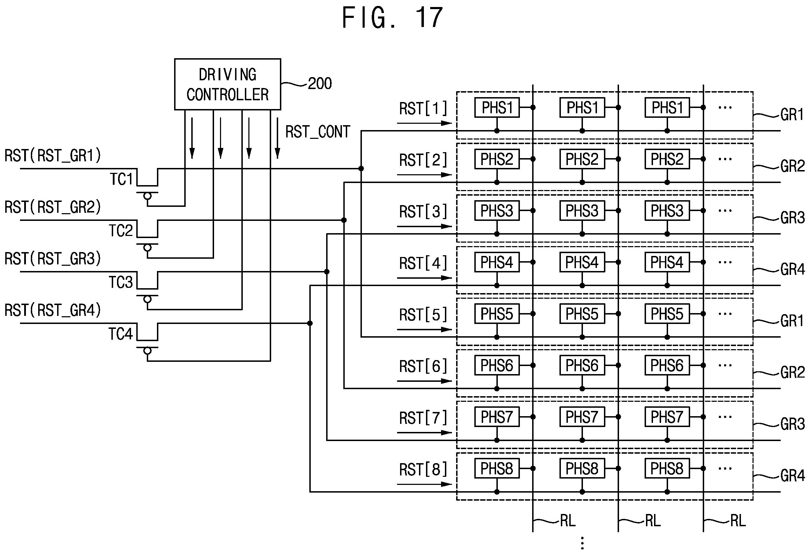

FIG. 17 is a diagram showing an example of a driving controller and optical sensing circuits of FIG. 16.

Referring to FIGS. 1 to 17, optical sensing circuits PHS according to embodiments of the present inventive concept may be grouped to form the first to n-th groups GR1 to GRn. During the n frames, the reset signals RST may be sequentially applied to the first to n-th groups GR1 to GRn as the first to n-th group reset signals. For example, in the embodiment of FIG. 17 where n = 4, optical sensing circuits PHS including first to eighth light sensing rows PHS1 to PHS8 may be grouped into first to fourth groups GR1 to GR4. The first group GR1 may include the first light sensing row PHS1 and the fifth light sensing row PHS5, the second group GR2 may include the second light sensing row PHS2 and the sixth light sensing row PHS6, the third group GR3 may include the third light sensing row PHS3 and the seventh light sensing row PHS7, and the fourth group GR4 may include the fourth light sensing row PHS4 and the eighth light sensing row PHS8.

The reset signal lines connected to the optical sensing circuits of the k-th group GRk and transmitting the k-th group reset signal may be connected to each other. Therefore, in the k-th frame, reset signals constituting the k-th group reset signal may be simultaneously applied to the optical sensing circuits included in the k-th group GRk. For example, reset signal lines connected to the light sensing rows PHS1, PHS5 of the first group GR1 may be connected to each other, and the first group reset signal RST_GR1 may be simultaneously applied as the reset signals RST[1], RST[5] to the light sensing rows PHS1, PHS5 of the first group GR1. For example, reset signal lines connected to the light sensing rows PHS2, PHS6 of the second group GR2 may be connected to each other, and the second group reset signal RST_GR2 may be simultaneously applied as the reset signals RST[2], RST[6] to the light sensing rows PHS2, PHS6 of the second group GR2. For example, reset signal lines connected to the light sensing rows PHS3, PHS7 of the third group GR3 may be connected to each other, and the third group reset signal RST_GR3 may be simultaneously applied to the light sensing rows PHS3, PHS7 of the third group GR3 as the reset signals RST[3], RST[7]. For example, reset signal lines connected to the light sensing rows PHS4, PHS8 of the fourth group GR4 may be connected to each other, and the fourth group reset signal RST_GR4 may be simultaneously applied to the light sensing rows PHS4, PHS8 of the fourth group GR4 as the reset signals RST[4], RST[8].

In an embodiment, a display device 10 may further include first to n-th control transistors, which are controlled based on a reset control signal RST_CONT output from a driving controller 200 and connected to the first to n-th groups GR1 to GRn. The first to n-th control transistors may sequentially provide the reset signals RST as the first to n-th group reset signals to the first to n-th groups GR1 to GRn in response to the reset control signal RST_CONT. The reset control signal RST_CONT may be included in a fourth control signal CONT4 output from the driving controller 200.

For example, the display device 10 may further include first to fourth control transistors TC1 to TC4, and the first to fourth control transistors TC1 to TC4 may be sequentially turned on in response to the reset control signal RST_CONT. Therefore, the first control transistor TC1 may be turned on in response to the reset control signal RST_CONT to simultaneously provide a first group reset signal RST_GR1 as the reset signal RST[1], RST[5] to the light sensing rows PHS1, PHS5 of the first group GR1. The second control transistor TC2 may be turned on in response to the reset control signal RST_CONT to simultaneously provide a second group reset signal RST_GR2 as the reset signal RST[2], RST[6] to the light sensing rows PHS2, PHS6 of the second group GR2. The third control transistor TC3 may be turned on in response to the reset control signal RST_CONT to simultaneously provide a third group reset signal RST_GR3 as the reset signal RST[3], RST[7] to the light sensing rows PHS3, PHS7 of the third group GR3. The fourth control transistor TC4 may be turned on in response to the reset control signal RST_CONT to simultaneously provide a fourth group reset signal RST_GR4 as the reset signal RST[4], RST[8] to the light sensing rows PHS4, PHS8 of the fourth group GR4.

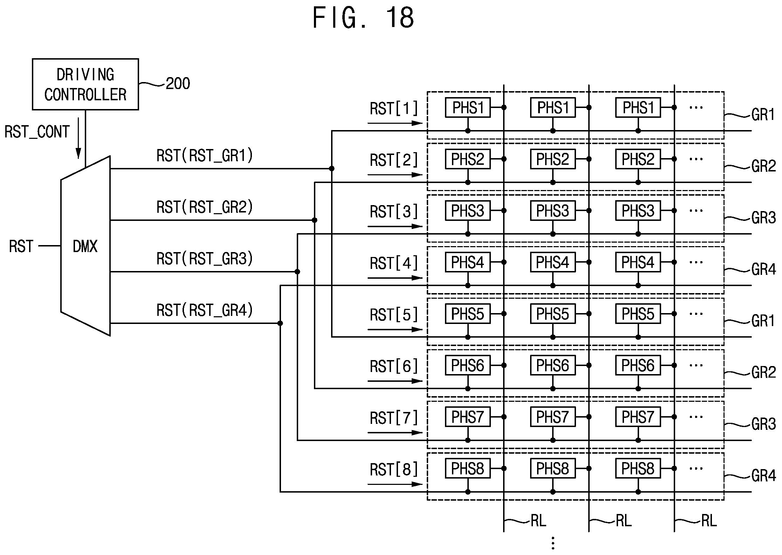

FIG. 18 is a diagram showing an example of a driving controller and optical sensing circuits of FIG. 16.

Referring to FIGS. 1 to 18, the optical sensing circuits PHS according to embodiments of the present inventive concept may be grouped to form the first to n-th groups GR1 to GRn. During the n frame, the reset signals RST may be sequentially applied to the first to n-th groups GR1 to GRn as the first to n-th group reset signals. For example, in the embodiment of FIG. 18 where n = 4, optical sensing circuits PHS including first to eighth light sensing rows PHS1 to PHS8 may be grouped into first to fourth groups GR1 to GR4. The first group GR1 may include the first light sensing row PHS1 and the fifth light sensing row PHS5, the second group GR2 may include the second light sensing row PHS2 and the sixth light sensing row PHS6, the third group GR3 may include the third light sensing row PHS3 and the seventh light sensing row PHS7, and the fourth group GR4 may include the fourth light sensing row PHS4 and the eighth light sensing row PHS8.

Reset signal lines connected to the optical sensing circuits of the k-th group GRk and transmitting the k-th group reset signal may be connected to each other. Therefore, in the k-th frame, reset signals constituting the k-th group reset signals may be simultaneously applied to the optical sensing circuits included in the k-th group GRk. For example, reset signal lines connected to the light sensing rows PHS1, PHS5 of the first group GR1 may be connected to each other, and the first group reset signal RST_GR1 may be simultaneously applied as the reset signals RST[1], RST[5] to the light sensing rows PHS1, PHS5 of the first group GR1. For example, reset signal lines connected to the light sensing rows PHS2, PHS6 of the second group GR2 may be connected to each other, and the second group reset signal RST_GR2 may be simultaneously applied as the reset signals RST[2], RST[6] to the light sensing rows PHS2, PHS6 of the second group GR2. For example, reset signal lines connected to the light sensing rows PHS3, PHS7 of the third group GR3 may be connected to each other, and the third group reset signal RST_GR3 may be simultaneously applied as the reset signals RST[3], RST[7] to the light sensing rows PHS3, PHS7 of the third group GR3. For example, reset signal lines connected to the light sensing rows PHS4, PHS8 of the fourth group GR4 may be connected to each other, and the fourth group reset signal RST_GR4 may be simultaneously applied as the reset signals RST[4], RST[8] to the light sensing rows PHS4, PHS8 of the fourth group GR4.

In an embodiment, a display device 10 may be controlled based on a reset control signal RST_CONT output from a driving controller 200, and may further include a demultiplexer (demux) DMX connected to the first to n-th groups GR1 to GRn. The demux DMX may sequentially provide the reset signals RST as the first to n-th group reset signals to the first to n-th groups GR1 to GRn in response to the reset control signal RST_CONT. The reset control signal RST_CONT may be included in a fourth control signal CONT4 output from the driving controller 200.

For example, the display device 10 may further include the demux DMX, and the demux DMX may sequentially provide the reset signals RST to the first to fourth groups GR1 to GR4 in response to the reset control signal RST_CONT. Therefore, the demux DMX may simultaneously provide a first group reset signal RST_GR1 as the reset signal RST[1], RST[5] to the light sensing rows PHS1, PHS5 of the first group GR1 in response to the reset control signal RST_CONT. The demux DMX may be turned on in response to the reset control signal RST_CONT to simultaneously provide a second group reset signal RST_GR2 as the reset signal RST[2], RST[6] to the light sensing rows PHS2, PHS6 of the second group GR2. The demux DMX may be turned on in response to the reset control signal RST_CONT to simultaneously provide a third group reset signal RST_GR3 as the reset signal RST[3], RST[7] to the light sensing rows PHS3, PHS7 of the third group GR3. The demux DMX may be turned on in response to the reset control signal RST_CONT to simultaneously provide a fourth group reset signal RST_GR4 as the reset signal RST[4], RST[8] to the light sensing rows PHS4, PHS8 of the fourth group GR4.

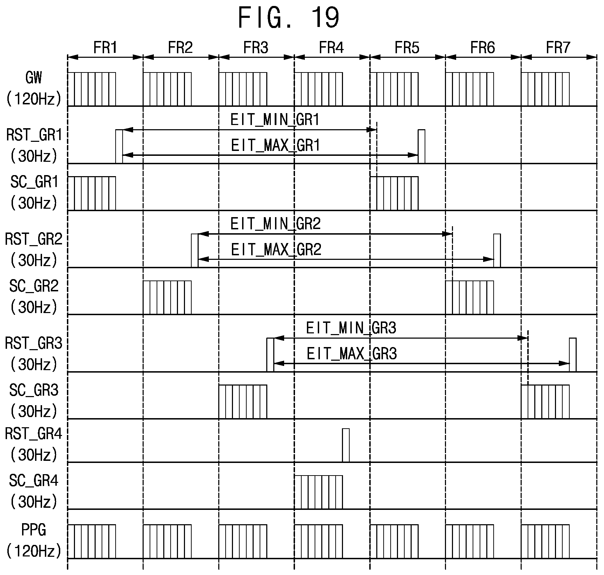

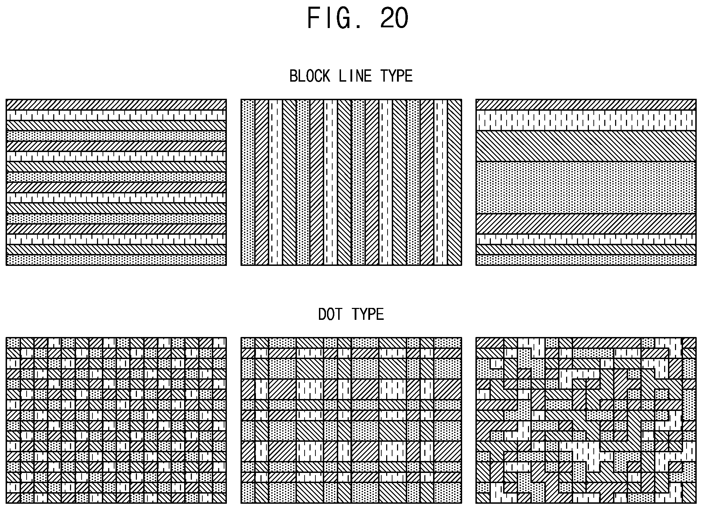

FIG. 19 is a timing diagram showing an example of signals applied to optical sensing circuits of FIG. 16 and FIG. 17, sensing currents generated by the optical sensing circuits of FIG. 16 and FIG. 17, and a digital sensing signal generated by a readout circuit of FIG. 1. FIG. 20 is a diagram showing a form of readout lines connected to optical sensing circuits of FIG. 16 and FIG. 17.

Referring to FIGS. 1 to 20, the driving frequency of the display panel 100 may be 120 Hz, and the frequency of the data write gate signal GW may be 120 Hz. One frame of 120 Hz may be 8.33 ms.

During the n frames, the reset signals RST may be sequentially applied to the first to n-th groups GR1 to GRn as the first to n-th group reset signals. In the k-th frame, reset signals constituting the k-th group reset signal may be simultaneously applied to the optical sensing circuits included in the k-th group GRk.

During the n frames, the data write gate signals GW may be sequentially applied to the first to n-th groups GR1 to GRn as first to n-th group data write gate signals. During a k-th frame, gate signals constituting a k-th group data write gate signal may be sequentially applied to optical sensing circuits included in the k-th group GRk.

During the n frames, first to n-th group sensing currents may be generated as the sensing currents SC in response to the data write gate signals GW. During the n frames, the digital sensing signal DSS may be generated based on the first to n-th group sensing currents.

A light exposure period EIT during which the optical sensing circuits PHS receive the light that is reflected off the user's body may be determined based on the k-th group reset signal and the k-th group data write gate signal. In an embodiment, a minimum light exposure period of the k-th group GRk may be than n-1 frame, and a maximum light exposure period of the k-th group GRk may be n frames.

For example, in a first frame FR1, the first group reset signal RST_GR1 may be simultaneously applied as the reset signals RST[1], RST[5] to the light sensing rows PHS1, PHS5 of the first group GR1. After the first group reset signal RST_GR1 is applied to the light sensing rows PHS1, PHS5 of the first group GR1, the light sensing rows PHS1, PHS5 of the first group GR1 may generate the photocharges in response to the light that is reflected by the user's body. During a fifth frame FR5, the data write gate signals GW[1], GW[5] may be sequentially applied as the first group data write gate signals to the light sensing rows PHS1, PHS5 of the first group GR1, and the light sensing rows PHS1, PHS5 of the first group GR1 may generate first group sensing currents SC_GR1 in response to the first group data write gate signals. For example, in the fifth frame FR5, the first group reset signal RST_GR1 may be simultaneously applied as the reset signals RST[1], RST[5] to the light sensing rows PHS1, PHS5 of the first group GR1. Therefore, a frequency of the first group reset signal RST_GR1 may be 30 Hz, and a frequency of the first group sensing currents SC_GR1 may be 30 Hz. A minimum light exposure period EIT_MIN of the first group GR1 may be 3 frames, and a maximum light exposure period EIT_MAX of the first group GR1 may be 4 frames.

For example, in a second frame FR2, the second group reset signal RST_GR2 may be simultaneously applied to the light sensing rows PHS2, PHS6 of the second group GR2 as the reset signals RST[2], RST[6]. After the second group reset signal RST_GR2 is applied to the light sensing rows PHS2, PHS6 of the second group GR2, the light sensing rows PHS2, PHS6 of the second group GR2 may generate the photocharges in response to the reflected light from the user's body. During a sixth frame FR6, the light of the second group GR2 may be the data write gate signals GW[2], GW[6] may be sequentially applied to the sensing rows PHS2, PHS6 as the second group data write gate signals, and the light sensing rows PHS2, PHS6 of the second group GR2 may generate second group sensing currents SC_GR2 in response to the second group data write gate signals. For example, in the sixth frame FR6, the second group reset signal RST_GR2 may be simultaneously applied as the reset signals RST[2], RST[6] to the light sensing rows PHS2, PHS6 of the second group GR2. Therefore, a frequency of the second group reset signal RST_GR2 may be 30 Hz, and a frequency of the second group sensing currents SC_GR2 may be 30 Hz. A minimum light exposure period EIT_MIN_GR2 of the second group GR2 may be 3 frames, and a maximum light exposure period EIT_MAX_GR2 of the second group GR2 may be 4 frames.

For example, in a third frame FR3, the third group reset signal RST_GR3 may be simultaneously applied to the light sensing rows PHS3, PHS7 of the third group GR3 as the reset signals RST[3], RST[7]. After the third group reset signal RST_GR3 is applied to the light sensing rows PHS3, PHS7 of the third group GR3, the light sensing rows PHS3, PHS7 of the third group GR3 may generate the photocharges in response to the reflected light from the user's body. During a seventh frame FR7, the data write gate signals GW[3], GW[7] may be sequentially applied as the third group data write gate signals to the light sensing rows PHS3, PHS7 of the third group GR3, and the light sensing rows PHS3, PHS7 of the third group GR3 may generate third group sensing currents SC_GR3 in response to the third group data write gate signals. For example, in the seventh frame FR7, the third group reset signal RST_GR3 may be simultaneously applied as the reset signals RST[3], RST[7] to the light sensing rows PHS3, PHS7 of the third group GR3. Therefore, a frequency of the third group reset signal RST_GR3 may be 30 Hz, and a frequency of the third group sensing currents SC_GR3 may be 30 Hz. A minimum light exposure period EIT_MIN of the third group GR3 may be 3 frames, and a maximum light exposure period EIT_MAX of the third group GR3 may be 4 frames.

For example, in a fourth frame FR4, the fourth group reset signal RST_GR4 may be simultaneously applied to the light sensing rows PHS4, PHS8 of the fourth group GR4 as the reset signals RST[4], RST[8]. After the fourth group reset signal RST_GR4 is applied to the light sensing rows PHS4, PHS8 of the fourth group GR4, the light sensing rows PHS4, PHS8 of the fourth group GR4 may generate the photocharges in response to the reflected light from the user's body. During an eighth frame FR8, the data write gate signals GW[4], GW[7] may be sequentially applied as the fourth group data write gate signals to the light sensing rows PHS4, PHS8 of the fourth group GR4, and the light sensing rows PHS4, PHS8 of the fourth group GR4 may generate fourth group sensing currents SC_GR4 in response to the fourth group data write gate signal. For example, in the eighth frame FR8, the fourth group reset signal RST_GR4 may be simultaneously applied as the reset signals RST[4], RST[8] to the light sensing rows PHS4, PHS8 of the fourth group GR4. Therefore, a frequency of the fourth group reset signal RST_GR4 may be 30 Hz, and a frequency of the fourth group sensing currents SC_GR4 may be 30 Hz. A minimum light exposure period EIT_MIN of the fourth group GR4 may be 3 frames, and a maximum light exposure period EIT_MAX of the fourth group GR4 may be 4 frames.

In the first frame FR1 and the fifth frame FR5, the first group sensing currents SC_GR1 may be converted into the digital sensing signal DSS, in the second frame FR2 and the sixth frame FR6, the second group sensing currents SC_GR2 may be converted into the digital sensing signal DSS, in the third frame FR3 and the seventh frame FR7, the third group sensing currents SC_GR3 may be converted into the digital sensing signal DSS, and in the fourth frame FR4 and the eighth frame, the fourth group sensing currents SC_GR4 may be converted into the digital sensing signal DSS. Therefore, a frequency of the digital sensing signal DSS (i.e., a pulse signal PPG) may be 120 Hz. The frequency of the digital sensing signal DSS may be equal to the driving frequency of the display panel 100.

As such, the optical sensing circuits PHS may be grouped to form first to n-th groups GR1 to GRn, and during the n frames, reset signals RST may be sequentially applied to the first to n-th groups GR1 to GRn as first to n-th group reset signals. Accordingly, the light exposure period EIT may be sufficiently secured, the light exposure period EIT may be constant at the upper and lower portions of the display panel 100, and the frequency of the digital sensing signal DSS (i.e., the pulse signal PPG) may be sufficiently secured. Therefore, the consistency and reliability of the digital sensing signal DSS may be secured. That is, an amplitude of the pulse signal PPG may be large, and the pulse signal PPS may be smooth.

In some embodiments, the first to n-th groups GR1 to GRn may be arranged in block line type shapes or dot type shapes (e.g. block line type groups or dot type groups). For example, in the block line type shapes, the readout lines RL may extend in the first direction. For example, in the block line type shapes, the readout lines RL may extend in the second direction intersecting the first direction. For example, in the block line type shapes, the number of readout lines included in each of the first to n-th groups GR1 to GRn may be different. For example, in dot type shapes, sizes of dots of the readout lines RL may be the same. For example, in the dot type shapes, the sizes of the dots of the readout lines RL may be different. For example, in the dot type shapes, the sizes of the dots of the readout lines RL may be non-uniform.

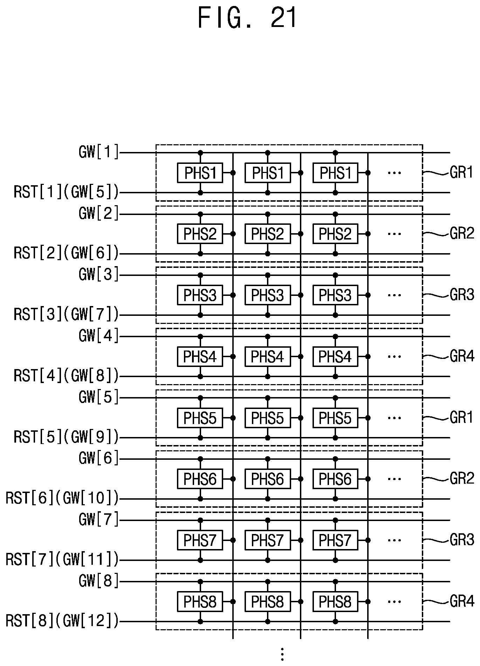

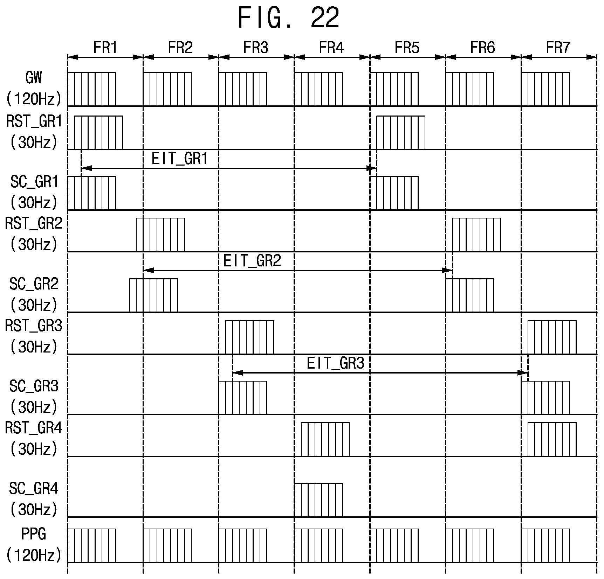

FIG. 21 is a diagram showing optical sensing circuits of FIG. 16 and data write gate signals and reset signals applied to the optical sensing circuits of FIG. 16. FIG. 22 is a timing diagram showing an example of signals applied to optical sensing circuits of FIG. 21, sensing currents generated by the optical sensing circuits of FIG. 21, and a digital sensing signal generated by a readout circuit of FIG. 1.

Referring to FIGS. 1 to 16, FIG. 21, and FIG. 22, the optical sensing circuits PHS according to embodiments of the present inventive concept may be grouped to form the first to n-th groups GR1 to GRn. During the n frame, the reset signals RST may be sequentially applied to the first to n-th groups GR1 to GRn as first to n-th group reset signals. For example, in the example of FIG. 21 where n = 4, optical sensing circuits PHS including first to eighth light sensing rows PHS1 to PHS8 may be grouped into first to fourth groups GR1 to GR4. The first group GR1 may include the first light sensing row PHS1 and the fifth light sensing row PHS5, the second group GR2 may include the second light sensing row PHS2 and the sixth light sensing row PHS6, the third group GR3 may include the third light sensing row PHS3 and the seventh light sensing row PHS7, and the fourth group GR4 may include the fourth light sensing row PHS4 and the eighth light sensing row PHS8.

During the n frames, data write gate signals GW may be sequentially applied to the first to n-th groups GR1 to GRn as first to n-th group data write gate signals. During a k-th frame, gate signals constituting a k-th group data write gate signal may be sequentially applied to optical sensing circuits included in the k-th group GRk.

The first to n-th group reset signals may be the first to n-th group data write gate signals. During the k-th frame, reset signals constituting a k-th group reset signal may be sequentially applied to the optical sensing circuits included in the k-th group.

A light exposure period EIT during which the optical sensing circuits PHS receive the light reflected by the user's body may be determined based on the k-th group reset signal and the k-th group data write gate signal. In an embodiment, a light exposure period of the k-th group GRk may be n frames.

When the optical sensing circuit PHS generates the sensing current SC in response to an i-th data write gate signal, the optical sensing circuit PHS may receive the i+n-th data write gate signal as the reset signal RST. For example, when a first optical sensing circuit PHS1 outputs a sensing current SC in response to a first data write gate signal GW[1], the first optical sensing circuit PHS1 may receive a fifth (=1+4) data write gate signal GW[5] as the reset signal RST[1]. For example, when a second optical sensing circuit PHS2 outputs a sensing current SC in response to a second data write gate signal GW[2], the second optical sensing circuit PHS2 may receive a sixth (=2+4) data write gate signal GW[6] as the reset signal RST[2]. For example, when a third optical sensing circuit PHS3 outputs a sensing current SC in response to a third data write gate signal GW[3], the third optical sensing circuit PHS1 may receive a seventh (=3+4) data write gate signal GW[7] as the reset signal RST[3]. For example, when a fourth optical sensing circuit PHS4 outputs a sensing current SC in response to a fourth data write gate signal GW[4], the fourth optical sensing circuit PHS4 may receive an eighth (=4+4) data write gate signal GW[8] as the reset signal RST[4].

For example, when a fifth optical sensing circuit PHS5 outputs a sensing current SC in response to a fifth data write gate signal GW[5], the fifth optical sensing circuit PHS5 may receive a ninth (=5+4) data write gate signal GW[9] as the reset signal RST[5]. For example, when a sixth optical sensing circuit PHS6 outputs a sensing current SC in response to a sixth data write gate signal GW[6], the sixth optical sensing circuit PHS6 may receive a tenth (=6+4) data write gate signal GW[10] as the reset signal RST[6]. For example, when a seventh optical sensing circuit PHS7 outputs a sensing current SC in response to a seventh data write gate signal GW[7], the seventh optical sensing circuit PHS7 may receive an eleventh (=7+4) data write gate signal GW[11] as the reset signal RST[7]. For example, when an eighth optical sensing circuit PHS8 outputs a sensing current SC in response to an eighth data write gate signal GW[8], the eighth optical sensing circuit PHS8 may receive a twelfth (=8+4) data write gate signal GW[12] as the reset signal RST[8].

For example, during a first frame FR1, the first group reset signal GW[5], GW[9] may be sequentially applied to the light sensing rows PHS1, PHS5 of the first group GR1. After the first group reset signal GW[5], GW[9] is sequentially applied to the light sensing rows PHS1, PHS5 of the first group GR1, the light sensing rows PHS1, PHS5 of the first group GR1 may generate the photocharges in response to the reflected light generated from the user's body. During a fifth frame FR5, the data write gate signals GW[1], GW[5], GW[9] may be sequentially applied as the first group data write gate signals to the light sensing rows PHS1, PHS5 of the first group GR1, and the light sensing rows PHS1, PHS5 of the first group GR1 may generate first group sensing currents SC_GR1 in response to the first group data write gate signals GW[1], GW[5], GW[9]. During the fifth frame FR5, the first group reset signal GW[5], GW[9] may be sequentially applied to the light sensing rows PHS1, PHS5 of the first group GR1. Therefore, a frequency of the first group reset signal RST_GR1 may be 30 Hz, and a frequency of the first group sensing currents SC_GR1 may be 30 Hz. A light exposure period EIT_GR1 of the first group GR1 may be 4 frames.

During a second frame FR2, the second group reset signal GW[6], GW[10] may be sequentially applied to the light sensing rows PHS2, PHS6 of the second group GR2. After the second group reset signal GW[6], GW[10] is sequentially applied to the light sensing rows PHS2, PHS6 of the second group GR2, the light sensing rows PHS2, PHS6 of the second group GR2 may generate the photocharges in response to the reflected light generated from the user's body. During a sixth frame FR6, the data write gate signals GW[2], GW[6], GW[10] may be sequentially applied as the second group data write gate signals to the light sensing rows PHS2, PHS6 of the second group GR2, and the light sensing rows PHS2, PHS6 of the second group GR2 may generate second group sensing currents SC_GR2 in response to the second group data write gate signals GW[2], GW[6], GW[10]. During the sixth frame FR6, the second group reset signal GW[6], GW[10] may be sequentially applied to the light sensing rows PHS2, PHS6 of the second group GR2. Therefore, a frequency of the second group reset signal RST_GR2 may be 30 Hz, and a frequency of the second group sensing currents SC_GR2 may be 30 Hz. A light exposure period EIT_GR2 of the second group GR2 may be 4 frames.

During a third frame FR3, the third group reset signal GW[7], GW[11] may be sequentially applied to the light sensing rows PHS3, PHS7 of the third group GR3. After the third group reset signal GW[7], GW[11] is sequentially applied to the light sensing rows PHS3, PHS7 of the third group GR3, the light sensing rows PHS3, PHS7 of the third group GR3 may generate the photocharges in response to the reflected light generated from the user's body. During a seventh frame FR7, the data write gate signals GW[3], GW[7], GW[11] may be sequentially applied as the third group data write gate signals to the light sensing rows PHS3, PHS7 of the third group GR3, and the light sensing rows PHS3, PHS7 of the third group GR3 may generate third group sensing currents SC_GR3 in response to the third group data write gate signals GW[3], GW[7], GW[11]. During the seventh frame FR7, the third group reset signal GW[7], GW[11] may be sequentially applied to the light sensing rows PHS3, PHS7 of the third group GR3. Therefore, a frequency of the third group reset signal RST_GR3 may be 30 Hz, and a frequency of the third group sensing currents SC_GR3 may be 30 Hz. A light exposure period EIT_GR3 of the third group GR3 may be 4 frames.

During a fourth frame FR4, the fourth group reset signal GW[8], GW[12] may be sequentially applied to the light sensing rows PHS4, PHS8 of the fourth group GR4. After the fourth group reset signal GW[8], GW[12] is sequentially applied to the light sensing rows PHS4, PHS8 of the fourth group GR4, the light sensing rows PHS4, PHS8 of the fourth group GR4 may generate the photocharges in response to the reflected light generated from the user's body. During an eighth frame FR8, the data write gate signals GW[4], GW[8], GW[12] may be sequentially applied as the fourth group data write gate signals to the light sensing rows PHS4, PHS8 of the fourth group GR4, and the light sensing rows PHS4, PHS8 of the fourth group GR4 may generate fourth group sensing currents SC_GR4 in response to the fourth group data write gate signals GW[4], GW[8], GW[12]. During the eighth frame FR8, the fourth group reset signal GW[8], GW[12] may be sequentially applied to the light sensing rows PHS4, PHS8 of the fourth group GR4. Therefore, a frequency of the fourth group reset signal RST_GR4 may be 30 Hz, and a frequency of the fourth group sensing currents SC_GR4 may be 30 Hz. A light exposure period EIT_GR4 of the fourth group GR4 may be 4 frames.

In the first frame FR1 and the fifth frame FR5, the first group sensing currents SC_GR1 may be converted into the digital sensing signal DSS, in the second frame FR2 and the sixth frame FR6, the second group sensing currents SC_GR2 may be converted into the digital sensing signal DSS, in the third frame FR3 and the seventh frame FR7, the third group sensing currents SC_GR3 may be converted into the digital sensing signal DSS, and in the fourth frame FR4 and the eighth frame, the fourth group sensing currents SC_GR4 may be converted into the digital sensing signal DSS. Therefore, a frequency of the digital sensing signal DSS may be 120 Hz. The frequency of the digital sensing signal DSS may be equal to the driving frequency of the display panel 100.

As such, the optical sensing circuits PHS may be grouped to form first to n-th groups GR1 to GRn, and during the n frames, reset signals RST may be sequentially applied to the first to n-th groups GR1 to GRn as first to n-th group reset signals. Accordingly, the light exposure period EIT may be sufficiently secured, the light exposure period EIT may be constant at the upper portion and the lower portion of the display panel 100, and the frequency of the digital sensing signal DSS (i.e., the pulse signal PPG) may be sufficiently secured. Therefore, the consistency and reliability of the digital sensing signal DSS may be secured. That is, an amplitude of the pulse signal PPG may be large, and the pulse signal PPG may be smooth.

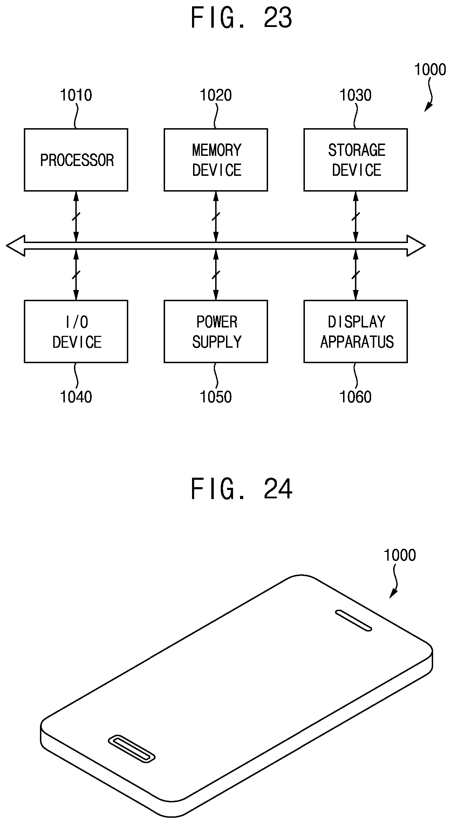

FIG. 23 is a block diagram showing an electronic device. FIG. 24 is a diagram showing an embodiment in which an electronic device of FIG. 23 is implemented as a smart phone.

Referring to FIGS. 23 and 24, an electronic device 1000 may include a processor 1010, a memory device 1020, a storage device 1030, an input/output I/O device 1040, a power supply 1050, and a display device 1060. The display device 1060 may be the display device 10 of FIG. 1. In addition, the electronic device 1000 may further include a plurality of ports for communicating with a video card, a sound card, a memory card, a universal serial bus USB device, other electronic device, and the like.

In an embodiment, as illustrated in FIG. 24, the electronic device 1000 may be implemented as the smart phone. However, the electronic device 1000 is not limited thereto. For example, the electronic device 1000 may be implemented as a cellular phone, a video phone, a smart pad, a smart watch, a tablet PC, a car navigation system, a computer monitor, a laptop, a head mounted display HMD device, and the like.

The processor 1010 may perform various computing functions. The processor 1010 may be a micro processor, a central processing unit CPU, an application processor AP, and the like. The processor 1010 may be coupled to other components via an address bus, a control bus, a data bus, and the like. Further, the processor 1010 may be coupled to an extended bus such as a peripheral component interconnection PCI bus.

The memory device 1020 may store data for operations of the electronic device 1000. For example, the memory device 1020 may include at least one nonvolatile memory device such as an erasable programmable read-only memory EPROM device, an electrically erasable programmable read-only memory EEPROM device, a flash memory device, a phase change random access memory PRAM device, a resistance random access memory RRAM device, a nano floating gate memory NFGM device, a polymer random access memory PoRAM device, a magnetic random access memory MRAM device, a ferroelectric random access memory FRAM device, and the like and/or at least one volatile memory device such as a dynamic random access memory DRAM device, a static random access memory SRAM device, a mobile DRAM device, and the like.

The storage device 1030 may include a solid state drive SSD device, a hard disk drive HDD device, a CD-ROM device, and the like.

The I/O device 1040 may include an input device such as a keyboard, a keypad, a mouse device, a touch-pad, a touch-screen, and the like, and an output device such as a printer, a speaker, and the like. In some embodiments, the I/O device 1040 may include the display device 1060.

The power supply 1050 may provide power for operations of the electronic device 1000.

The display device 1060 may be connected to other components through buses or other communication links.

The inventive concepts may be applied to any display device and any electronic device including the touch panel. For example, the inventive concepts may be applied to a mobile phone, a smart phone, a tablet computer, a digital television TV, a 3D TV, a personal computer PC, a home appliance, a laptop computer, a personal digital assistant PDA, a portable multimedia player PMP, a digital camera, a music player, a portable game console, a navigation device, etc.

Embodiments are set out in the following clauses:
Clause 1. A display device, comprising: a display panel including pixel circuits and optical sensing circuits; a gate driver configured to provide gate signals to the pixel circuits and the optical sensing circuits; a readout circuit receiving sensing currents output from the optical sensing circuits in response to the gate signals and generating a pulse signal based on the sensing currents; and a driving controller configured to control the gate driver and the readout circuit, and to determine a user's a biometric indicator based on the pulse signal, wherein the optical sensing circuits initialized with a reset voltage in response to reset signals are grouped into first to n-th groups, and wherein the reset signals are sequentially applied to the first to n-th groups as first to n-th group reset signals during n frames, wherein n is an integer greater than or equal to 2.
Clause 2. The display device of clause 1, wherein the display panel includes a display region in which the pixel circuits emit light and a sensing region in which the optical sensing circuits receive light that is reflected by the user, wherein the sensing region is the display region.
Clause 3. The display device of clause 1 or 2 wherein, during the n frames, the gate signals are sequentially applied to the first to n-th groups as the first to n-th group gate signals, and during a k-th frame, the gate signals constituting a k-th group gate signal are sequentially applied to optical sensing circuits included in a k-th group, wherein k is an integer greater than or equal to 1 and less than or equal to n.
Clause 4. The display device of clause 3 wherein, during the n frames, first to n-th group sensing currents are generated as the sensing currents in response to the gate signals, and wherein during the n frames, the pulse signal is generated based on the first to n-th group sensing currents.
Clause 5. The display device of clause 4, wherein a frequency of the pulse signal is equal to a driving frequency of the display panel.
Clause 6. The display device of clause 4 or 5 wherein, in the k-th frame, reset signals constituting the k-th group reset signal are simultaneously applied to the optical sensing circuits included in the k-th group.
Clause 7. The display device of clause 6, wherein reset signal lines connected to the optical sensing circuits of the k-th group and transmitting the k-th group reset signal are connected to each other.
Clause 8. The display device of clause 6 or 7, wherein a light exposure period in which the optical sensing circuits receive reflected light from the user is determined based on a k-th group reset signal and a k-th group gate signal, and a light exposure period of the k-th group is greater than n-1 frames and less than or equal to n frames.
Clause 9. The display device of any one of clauses 6 to 8, wherein the display device further comprises first to n-th control transistors connected to the first to n-th groups and controlled based on a reset control signal output from the driving controller, and the first to n-th control transistors sequentially provide the reset signals to the first to n-th groups as the first to n-th group reset signals in response to the reset control signal.
Clause 10. The display device of any one of clauses 6 to 9, wherein the display device further comprises a demultiplexer connected to the first to n-th groups and controlled based on a reset control signal output from the driving controller, and wherein the demux sequentially provides the reset signals to the first to n-th groups as the first to n-th group reset signals in response to the reset control signal.
Clause 11. The display device of any one of clauses 6 to 10, wherein the first to n-th groups are grouped to form block line type groups or a dot type groups.
Clause 12. The display device of any one of clauses 4 to 11, wherein the first to n-th group reset signals are equal to the first to n-th group gate signals, and wherein, during the k-th frame, the reset signals constituting the k-th group reset signal are sequentially applied to the optical sensing circuits in the k-th group.
Clause 13. The display device of clause 12, wherein, when the optical sensing circuit outputs the sensing current in response to the i-th gate signal, the optical sensing circuit receives the i+n-th gate signal as the reset signal, wherein i is an integer greater than or equal to 2.
Clause 14. The display device of clause 13, wherein the light exposure period during which the optical sensing circuits receive a reflected light from the user is determined based on the k-th group reset signal and the k-th group gate signal, and wherein the light exposure period is n frames long.
Clause 15. The display device of any one of clauses 4 to 14, wherein a light exposure period during which the optical sensing circuits receive a reflected light from the user is determined based on a k-th group reset signal and the k-th group gate signal, and wherein, an amplitude of the pulse signal increases with the light exposure period.
Clause 16. The display device of any preceding clause, wherein, when a frequency of the pulse signal is high, a graph of the pulse signal appears thick due to a high number of jagged portions.
Clause 17. The display device of any preceding clause, wherein the readout circuit comprises: an integrator including an inverting input terminal connected to the readout line, a non-inverting input terminal receiving a reference voltage, and an output terminal, and an integrating capacitor connected between the inverting input terminal of the amplifier and the output terminal of the amplifier, and integrating the sensing current to generate a signal voltage; a signal capacitor accumulating and storing the signal voltage; and an analog-to-digital converter connected to the signal capacitor.
Clause 18. The display device of any preceding clause, wherein the display device further comprises a data driver configured to provide a data voltage to the pixel circuits, and wherein
each of the pixel circuits comprises: a first pixel transistor which generates a driving current; a second pixel transistor which provides the data voltage to the first pixel transistor in response to a data write gate signal; and a light emitting element which emits light based on the driving current.
Clause 19. The display device of clause 18, wherein the display device further comprises an emission driver configured to provide an emission signal to the pixel circuits, wherein each of the pixel circuits further comprises: a third pixel transistor diode-connecting the first pixel transistor in response to a compensation gate signal; a fourth pixel transistor providing a first initialization voltage to a gate electrode of the first pixel transistor in response to a data initialization gate signal; fifth and sixth pixel transistors applying the driving current to the light emitting element in response to the emission signal; and a seventh pixel transistor providing a second initialization voltage to a first electrode of the light emitting element in response to a light emitting element initialization gate signal, and wherein each of the optical sensing circuits comprises: a first sensing transistor which generates the sensing current based on a voltage of a first electrode of the sensing element; a second sensing transistor which provides the sensing current to the readout line in response to the data write gate signal; and a third sensing transistor which provides the reset voltage to the first electrode of the sensing element in response to the reset signal.
Clause 20. An electronic device, comprising: a display panel including pixel circuits and optical sensing circuits; a gate driver configured to provide gate signals to the pixel circuits and the sensing circuits; a readout circuit receiving sensing currents output from the optical sensing circuits in response to the gate signals and generating a pulse signal based on the sensing currents; a driving controller configured to control the gate driver and the readout circuit, and to determine a user's a biometric indicator based on the pulse signal; and one or more processors configured to control the driving controller, wherein the optical sensing circuits initialized with a reset voltage in response to reset signals are grouped to include first to n-th groups, and wherein the reset signals are sequentially applied to the first to n-th groups as first to n-th group reset signals during n frames, where n is an integer greater than or equal to 2.

The foregoing is illustrative of the inventive concept and is not to be construed as limiting thereof. Although a few embodiments of the inventive concept have been described, those skilled in the art will readily appreciate that many modifications are possible in the embodiments without materially departing from the novel teachings and advantages of the inventive concept. Accordingly, all such modifications are intended to be included within the scope of the inventive concept as defined in the claims. In the claims, means-plus-function clauses are intended to cover the structures described herein as performing the recited function and not only structural equivalents but also equivalent structures. Therefore, it is to be understood that the foregoing is illustrative of the inventive concept and is not to be construed as limited to the specific embodiments disclosed, and that modifications to the disclosed embodiments, as well as other embodiments, are intended to be included within the scope of the appended claims. The inventive concept is defined by the following claims.

## Claims

1. A display device (10), comprising:
a display panel (100) including pixel circuits (PX) and optical sensing circuits (PHS);
a gate driver (300) configured to provide gate signals (GS) to the pixel circuits (PX) and the optical sensing circuits (PHS);
a readout circuit (700) configured to receive sensing currents (SC) output from the optical sensing circuits (PHS) in response to the gate signals (GS) and to generate a pulse signal (PPG) based on the sensing currents (SC); and
a driving controller (200) configured to control the gate driver (300) and the readout circuit (700), and to determine a user's biometric indicator based on the pulse signal (PPG),
wherein the optical sensing circuits (PHS) initialized with a reset voltage (VRST) in response to reset signals (RST) are grouped into first to n-th groups, and
wherein the reset signals (RST) are sequentially applied to the first to n-th groups as first to n-th group reset signals during n frames, wherein n is an integer greater than or equal to 2.

2. The display device of claim 1, wherein the display panel (100) includes a display region (DR) in which the pixel circuits (PX) emit light and a sensing region (SR) in which the optical sensing circuits (PHS) receive light that is reflected by the user,
wherein the sensing region (SR) is the display region (DR).

3. The display device of claim 1 or 2 wherein, during the n frames, the gate signals (GS) are sequentially applied to the first to n-th groups as the first to n-th group gate signals, and
during a k-th frame, the gate signals (GS) constituting a k-th group gate signal are sequentially applied to optical sensing circuits (PHS) included in a k-th group, wherein k is an integer greater than or equal to 1 and less than or equal to n.

4. The display device of claim 3 wherein, during the n frames, first to n-th group sensing currents (SC) are generated as the sensing currents (SC) in response to the gate signals (GS), and
wherein during the n frames, the pulse signal (PPG) is generated based on the first to n-th group sensing currents (SC), and
wherein a frequency of the pulse signal (PPG) is optionally equal to a driving frequency of the display panel (100).

5. The display device of claim 4 wherein, in the k-th frame, reset signals (RST) constituting the k-th group reset signal are simultaneously applied to the optical sensing circuits (PHS) included in the k-th group, wherein reset signal lines connected to the optical sensing circuits (PHS) of the k-th group and transmitting the k-th group reset signal are optionally connected to each other.

6. The display device of claim 5, wherein a light exposure period (EIT) in which the optical sensing circuits (PHS) receive reflected light from the user is determined based on a k-th group reset signal and a k-th group gate signal, and
a light exposure period (EIT) of the k-th group is greater than n-1 frames and less than or equal to n frames.

7. The display device of any one of claims 5 to 6, wherein the display device further comprises first to n-th control transistors (TC1-TC4) connected to the first to n-th groups and controlled based on a reset control signal (RST_CONT) output from the driving controller (200), and
the first to n-th control transistors (TC1-TC4) sequentially provide the reset signals (RST) to the first to n-th groups as the first to n-th group reset signals in response to the reset control signal (RST_CONT),
and/or wherein the display device further comprises a demultiplexer (DMX) connected to the first to n-th groups and controlled based on a reset control signal (RST_CONT) output from the driving controller (200), and
wherein the demux (DMX) sequentially provides the reset signals (RST) to the first to n-th groups as the first to n-th group reset signals in response to the reset control signal (RST_CONT).

8. The display device of any one of claims 5 to 7, wherein the first to n-th groups are grouped to form block line type groups or a dot type groups.

9. The display device of any one of claims 4 to 8, wherein the first to n-th group reset signals are equal to the first to n-th group gate signals, and
wherein, during the k-th frame, the reset signals (RST) constituting the k-th group reset signal are sequentially applied to the optical sensing circuits (PHS) in the k-th group.

10. The display device of claim 9, wherein, when the optical sensing circuit (PHS) outputs the sensing current (SC) in response to the i-th gate signal, the optical sensing circuit (PHS) receives the i+n-th gate signal as the reset signal (RST), wherein i is an integer greater than or equal to 2,
wherein optionally, the light exposure period (EIT) during which the optical sensing circuits (PHS) receive a reflected light from the user is determined based on the k-th group reset signal and the k-th group gate signal, and wherein the light exposure period (EIT) is n frames long.

11. The display device of any one of claims 4 to 10, wherein a light exposure period (EIT) during which the optical sensing circuits (PHS) receive a reflected light from the user is determined based on a k-th group reset signal and the k-th group gate signal, and
wherein, an amplitude of the pulse signal (PPG) increases with the light exposure period (EIT).

12. The display device of any preceding claim, wherein the readout circuit (700) comprises:
an integrator comprising an amplifier (AMP) including an inverting input terminal connected to the readout line (RL), a non-inverting input terminal receiving a reference voltage (VREF), and an output terminal, and an integrating capacitor (ICAP) connected between the inverting input terminal of the amplifier (AMP) and the output terminal of the amplifier (AMP), and integrating the sensing current (SC) to generate a signal voltage;
a signal capacitor (SCAP) accumulating and storing the signal voltage; and
an analog-to-digital converter (ADC) connected to the signal capacitor (SCAP),
and/or wherein, when a frequency of the pulse signal (PPG) is high, a graph of the pulse signal (PPG) appears thick due to a high number of jagged portions.

13. The display device of any preceding claim, wherein the display device further comprises a data driver (500) configured to provide a data voltage (VDATA) to the pixel circuits (PX), and
wherein each of the pixel circuits (PX) comprises:
a first pixel transistor (PT1) which generates a driving current;
a second pixel transistor (PT2) which provides the data voltage (VDATA) to the first pixel transistor (PT1) in response to a data write gate signal (GW); and
a light emitting element (EE) which emits light based on the driving current.

14. The display device of claim 13, wherein the display device further comprises an emission driver (600) configured to provide an emission signal (EM) to the pixel circuits (PX),
wherein each of the pixel circuits (PX) further comprises:
a third pixel transistor (PT3) diode-connecting the first pixel transistor (PT1) in response to a compensation gate signal (GC);
a fourth pixel transistor (PT4) providing a first initialization voltage (VINT1) to a gate electrode of the first pixel transistor (PT1) in response to a data initialization gate signal (GI);
fifth (PT5) and sixth (PT6) pixel transistors applying the driving current to the light emitting element (EE) in response to the emission signal (EM); and
a seventh pixel transistor (PT7) providing a second initialization voltage (VINT2) to a first electrode of the light emitting element in response to a light emitting element initialization gate signal (GB), and
wherein each of the optical sensing circuits (PHS) comprises:
a first sensing transistor (ST1) which generates the sensing current (SC) based on a voltage of a first electrode of a sensing element (PD);
a second sensing transistor (ST2) which provides the sensing current (SC) to the readout line (RL) in response to the data write gate signal (GW); and
a third sensing transistor (ST3) which provides the reset voltage (VRST) to the first electrode of the sensing element (PD) in response to the reset signal (RST).

15. An electronic device (1000), comprising:
the display panel (100) of any preceding claim; and
one or more processors (1010) configured to control the driving controller (200).
